# EUROPEAN PATENT APPLICATION

(11) **EP 3 992 111 A1**
(43) Date of publication of application: **04.05.2022**
(21) Application number: 20832366.7
(22) Date of filing: 22.06.2020
(51) Int. Cl.: B65D 90/04, B60P 3/04, B65D 88/12

(54) **LIVESTOCK TRANSPORTATION CONTAINER**

(30) Priority: 25.06.2019 JP 2019117507
(71) Applicant: Mitsubishi Materials Corporation, Tokyo 100-8117 (JP)
(72) Inventor: SAKAMOTO Toshio, Kitamoto-shi, Saitama 364-0028 (JP); KATO Komei, Saitama-shi, Saitama 330-8508 (JP)
(74) Representative: Hoffmann Eitle
(86) International application number: PCT/JP2020/024468
(87) International publication number: WO 2020/262324

(57) **Abstract**

A livestock transportation container (10) is used to house and transport livestock, and includes a copper layer (20) configured to be made of copper or a copper alloy and provided on at least a part of an inner surface of a housing chamber (11) in which the livestock is housed. The copper layer (20) is preferably provided so as to occupy 10% or more of a total area of the inner surface of the housing chamber (11).

## Description

### [Technical Field]

The present invention relates to a livestock transportation container which is used to house and transport various livestock such as cattle, horses, pigs, sheep, and birds.

Priority is claimed on Japanese Patent Application No. 2019-117507, filed June 25, 2019, the content of which is incorporated herein by reference.

### [Background Art]

Measures against various epidemics have been implemented in order to maintain a good health status of livestock reared in the livestock industry. For example, Patent Document 1 proposes, as means for suppressing the invasion of pathogens such as bacteria and viruses in livestock rearing farms and the spread of infection, a feed to which a photocatalyst material containing apatite having photocatalytic activity is added, a rearing jig having the above-described photocatalyst material, a rearing facility having the above-described photocatalyst material, and the like.

In addition, in the livestock industry, livestock may be moved between rearing farms for breeding, or moved to slaughterhouses for meat processing.

In the transportation of the above-described livestock, a livestock transportation container provided with a housing chamber which houses livestock is used. For example, in Patent Documents 2 and 3, a livestock transportation vehicle provided with a load carrying platform (livestock transportation container) for housing livestock is widely used.

In addition, in the transportation of the above-described livestock by an aircraft, a ship, or the like, for example, a container for livestock transportation (livestock transportation container) which houses livestock is used.

Here, in the transportation of livestock, it is necessary to reduce stress on the livestock and maintain a health status.

Therefore, in the livestock transportation vehicle described in Patent Document 3, in order to keep a housing chamber for the livestock to be housed clean and to adjust a temperature in the chamber, a water spray pipe is provided at a position higher than that of the livestock to be housed, water passing means is provided in a floor surface of the load carrying platform, and a waste water tank is provided below the floor surface of the load carrying platform.

### [Citation List]

### [Patent Documents]

[Patent Document 1]
   Japanese Unexamined Patent Application, First Publication No. 2012-165758
[Patent Document 2]
   Japanese Unexamined Patent Application, First Publication No. 2001-047868
[Patent Document 3]
   Japanese Unexamined Patent Application, First Publication No. 2002-087148

### [Summary of Invention]

### [Technical Problem]

In recent years, highly infectious epidemics such as foot-and-mouth disease, classical swine fever, and bird flu have spread to various livestock and have become a big problem.

Here, since bacteria and viruses that are pathogens of the above-described epidemics have extremely high infectivity, there is concern that the invasion of pathogens and the spread of contamination may not be sufficiently suppressed even in a case where a feed, a rearing jig, and a rearing facility containing a photocatalyst material are used as shown in Patent Document 1.

In addition, in a case where the above-described epidemics occur, the movement of livestock around the area where the epidemic occurred is restricted to prevent the spread to other areas. Furthermore, even in a case where livestock is transported outside the movement restriction area, measures such as selecting a transportation route to avoid other rearing farms are implemented.

However, in the transportation of livestock, since the livestock is housed in a relatively narrow housing chamber, there is concern that the spread of epidemics may not be sufficiently prevented even in a case where the above-described measures are implemented against the epidemics caused by pathogens having extremely high infectivity. In addition, in a case where pathogens invade the housing chamber from the outside during the transportation, it is necessary to rapidly kill the pathogens in order to prevent the infection of the livestock in the housing chamber.

Therefore, in the transportation of livestock, it is required to suppress the spread of pathogens such as bacteria and viruses more than ever before.

The present invention is contrived on the background of the above circumstances, and an objective thereof is to provide a livestock transportation container capable of suppressing the spread of pathogens such as bacteria and viruses to livestock housed in a housing chamber even in the transportation of the livestock, and maintaining a health status of the livestock to be transported.

### [Solution to Problem]

The inventors have conducted intensive studies to solve the problems, and found that a copper member made of copper and a copper alloy has a higher action speed on pathogens than the above-described photocatalyst material, has particularly excellent antibacterial properties and antiviral properties, and can efficiently suppress the spread of pathogens even in the transportation of livestock.

The present invention is made based on the above findings, and a livestock transportation container according to the present invention is a livestock transportation container which is used to house and transport livestock, the livestock transportation container including a copper layer made of copper or a copper alloy and provided on at least a part of an inner surface of a housing chamber for the livestock to be housed.

In the livestock transportation container having the above configuration, since the copper layer made of copper or a copper alloy having a high action speed on pathogens and having excellent antibacterial properties and antiviral properties is provided on at least a part of the inner surface of the housing chamber for the livestock to be housed, it is possible to suppress the spread of pathogens to the livestock housed in the housing chamber. Accordingly, it is possible to transport the livestock while maintaining a good health status of the livestock.

Here, in the livestock transportation container according to the present invention, the copper layer is preferably provided so as to occupy 10% or more of a total area of the inner surface.

In this case, since the copper layer occupies 10% or more of the total area of the inner surface of the housing chamber for the livestock to be housed, pathogens can be efficiently killed, and it is possible to further suppress the spread of pathogens to the livestock in the housing chamber.

In addition, in the livestock transportation container according to the present invention, the copper layer preferably has a thickness in a range of 1 µm or more and less than 10 mm.

In this case, since the thickness of the copper layer is 1 µm or more, the antibacterial properties and the antiviral properties can be maintained. Since the thickness of the copper layer is less than 10 mm, an excessive increase of the weight of the housing chamber can be suppressed.

Furthermore, in the livestock transportation container according to the present invention, the copper layer may have a Cu content of 40 mass% or more.

In this case, since the Cu content is 40 mass% or more, the Cu content is secured, the antibacterial action and the antiviral action can be sufficiently exerted, and it is possible to suppress the spread of pathogens.

In addition, in the livestock transportation container according to the present invention, the copper layer may have an Ag content in a range of 5 mass ppm or more and 1 mass% or less.

In this case, since the Ag content is 5 mass ppm or more, pathogens can be killed by the antibacterial action and the antiviral action by Ag ions. Since the Ag content is 1 mass% or less, the content of expensive Ag can be suppressed, and the manufacturing cost can be reduced.

Furthermore, in the livestock transportation container according to the present invention, the copper layer preferably has a Cu content in a range of 52 mass% or more and 56 mass% or less, an Ni content in a range of 10 mass% or more and 12 mass% or less, a Mn content in a range of 0.1 mass% or more and 1.0 mass% or less, and a Zn balance containing inevitable impurities.

In this case, since the copper layer has the above-described composition, particularly excellent antibacterial properties and antiviral properties are obtained, and it is possible to further suppress the spread of pathogens. In addition, deterioration of appearance can be suppressed even after long-term use due to excellent color fastness.

In addition, in the livestock transportation container according to the present invention, the copper layer preferably has a Cu content in a range of 69 mass% or more and 73 mass% or less, an Ni content in a range of 1.5 mass% or more and 3.5 mass% or less, a Sn content in a range of 0.3 mass% or more and 0.9 mass% or less, a P content in a range of 0.01 mass% or more and 0.03 mass% or less, and a Zn balance containing inevitable impurities.

In this case, since the copper layer has the above-described composition, particularly excellent antibacterial properties and antiviral properties are obtained, and it is possible to further suppress the spread of pathogens. In addition, deterioration of appearance can be suppressed even after long-term use due to excellent color fastness.

Furthermore, in the livestock transportation container according to the present invention, the copper layer is preferably also provided on at least a part of an outer surface of the housing chamber.

In this case, even pathogens such as bacteria and viruses adhering to the outer surface of the housing chamber can be killed by the antibacterial action and the antiviral action of the copper layer, and it is possible to sufficiently suppress the invasion of pathogens from the outside.

In addition, in the livestock transportation container according to the present invention, the copper layer is preferably provided so as to occupy 10% or more of a total area of the outer surface.

In this case, since the copper layer occupies 10% or more of the total area of the outer surface of the housing chamber for the livestock to be housed, pathogens adhering to the outer surface of the housing chamber can be efficiently killed, and it is possible to further suppress the invasion of pathogens from the outside.

Furthermore, in the livestock transportation container according to the present invention, the copper layer may be provided so as to occupy 10% or more of an area of a floor surface in the inner surface.

In this case, since the copper layer is provided so as to occupy 10% or more of the area of the floor surface in the inner surface, pathogens can be killed by the antibacterial action and the antiviral action of the copper layer even in a case where pigs or the like touch tips of their noses to the floor surface, and it is possible to further suppress the spread of the pathogens.

Alternatively, in the livestock transportation container according to the present invention, the copper layer may be provided so as to occupy 10% or more of an area of a floor surface and a side surface from the floor surface to a height of 50 cm in the inner surface.

In this case, since the copper layer is provided so as to occupy 10% or more of the area of the floor surface and the side surface from the floor surface to a height of 50 cm in the inner surface, pathogens can be killed by the antibacterial action and the antiviral action of the copper layer even in a case where pigs or the like touch tips of their noses to the floor surface or the region in the side surface from the floor surface to a height of 50 cm, and it is possible to further suppress the spread of the pathogens.

### [Advantageous Effects of Invention]

According to the present invention, it is possible to provide a livestock transportation container capable of suppressing the spread of pathogens such as bacteria and viruses to livestock housed in a housing chamber even in the transportation of the livestock, and maintaining a health status of the livestock to be transported.

### [Brief Description of Drawings]

Fig. 1 is an explanatory diagram showing an example of a livestock transportation vehicle provided with a livestock transportation container (load carrying platform for livestock transportation) according to the embodiment of the present invention.
Fig. 2 is a vertical cross-sectional view of the livestock transportation container shown in Fig. 1.
Fig. 3 is a cross-sectional view taken along the line X-X in Fig. 1.

### [Description of Embodiments]

Hereinafter, a livestock transportation container according to an embodiment of the present invention will be described.

The livestock transportation container according to this embodiment is used as, for example, a load carrying platform of a livestock transportation vehicle. Examples of livestock to be transported include cattle, horses, pigs, sheep, and birds.

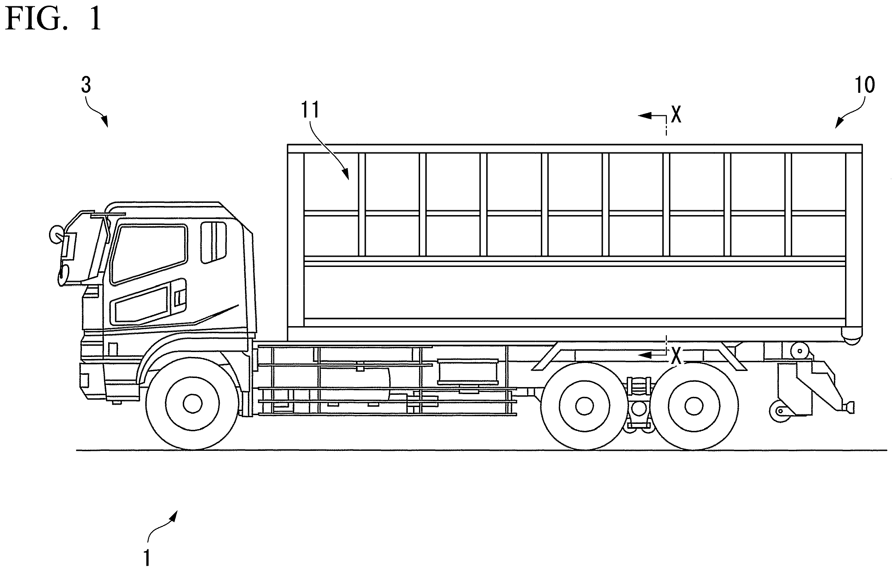

Fig. 1 shows a livestock transportation vehicle 1 provided with a livestock transportation container (load carrying platform for livestock transportation) according to an embodiment of the present invention.

The livestock transportation vehicle 1 includes a livestock transportation container 10 according to this embodiment and a transportation vehicle 3 on which the livestock transportation container 10 can be mounted.

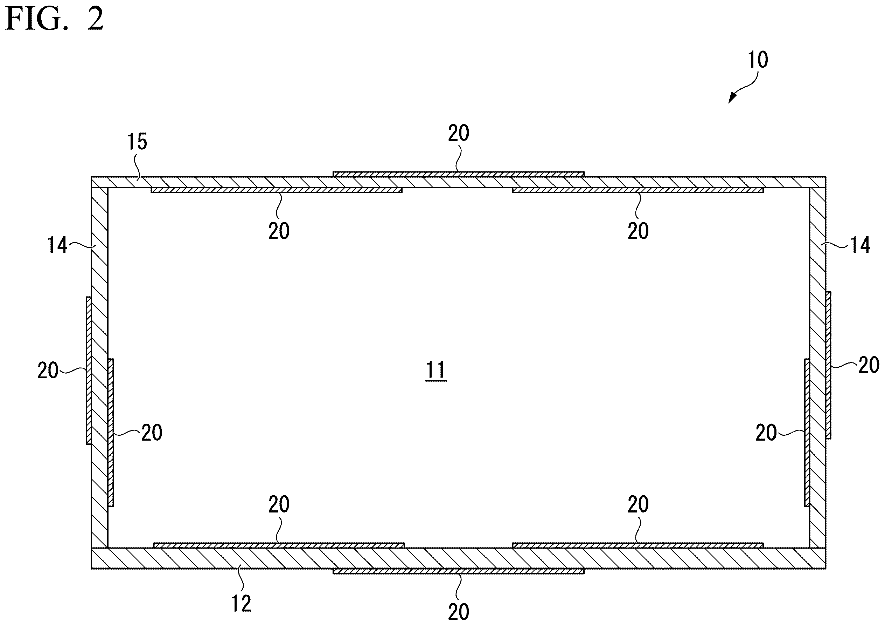

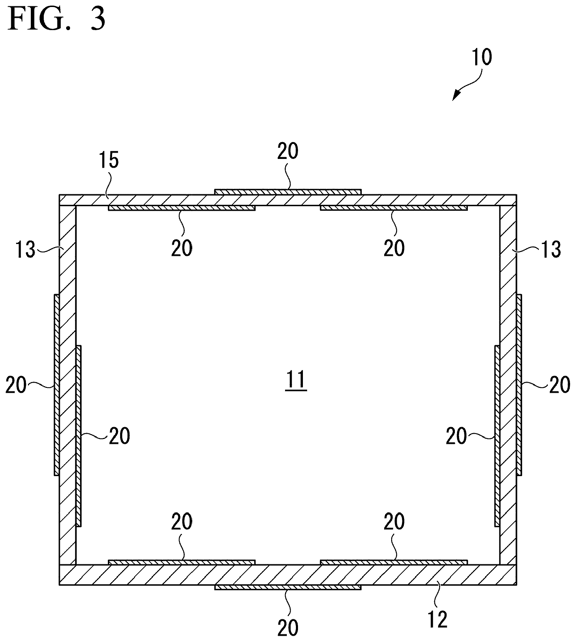

As shown in Figs. 1 to 3, the livestock transportation container 10 according to the embodiment of the present invention is provided with a housing chamber 11 which houses livestock, and the housing chamber 11 has a structure surrounded by a floor surface 12, a side wall portion 13, an end face portion 14, and a ceiling portion 15.

The side wall portion 13 has a lower portion as a panel portion and an upper portion as a fence portion, and is configured so that outside air can be taken into the housing chamber 11 through the fence portion.

In the livestock transportation container 10 according to this embodiment, a copper layer 20 made of copper or a copper alloy is provided on at least a part of an inner surface of the housing chamber 11. In this embodiment, as described above, since the housing chamber 11 has a structure surrounded by the floor surface 12, the side wall portion 13, the end face portion 14, and the ceiling portion 15, the copper layer 20 is provided on at least a part of the inner surface side of the floor surface 12, the side wall portion 13, the end face portion 14, and the ceiling portion 15.

The copper layer 20 made of copper or a copper alloy has excellent antibacterial properties and antiviral properties, and can suppress the spread of pathogens.

Since livestock excretion may be accumulated on the floor surface 12, the copper layer 20 may not be provided. However, since pigs or the like rub tips of their noses on the floor surface 12, the copper layer 20 is preferably also provided on the floor surface 12. As above, a position of the copper layer 20 to be provided is preferably set according to the livestock to be transported.

Here, in this embodiment, the above-described copper layer 20 is preferably provided so as to occupy 10% or more of the total area of the inner surface of the housing chamber 11.

The above-described copper layer 20 preferably occupies 20% or more, and more preferably 30% or more of the total area of the inner surface of the housing chamber 11. Although not particularly limited, the above-described copper layer 20 may be provided so as to occupy 10% or more and 100% or less or 98% or less of the total area of the inner surface of the housing chamber 11.

Furthermore, in this embodiment, in a case where livestock such as pigs whose tips of noses touch the floor surface is transported, the copper layer 20 may be provided so as to occupy 10% or more of the area of the floor surface in the inner surface of the housing chamber 11. Alternatively, the copper layer 20 may be provided so as to occupy 10% or more of the area of the floor surface and the side surface from the floor surface to a height of 50 cm in the inner surface of the housing chamber 11.

The above-described copper layer 20 preferably occupies 20% or more, and more preferably 30% or more of the area of the floor surface in the inner surface of the housing chamber 11. Although not particularly limited, the above-described copper layer 20 may occupy 10% or more and 100% or less or 98% or less of the area of the floor surface in the inner surface of the housing chamber 11. In addition, the copper layer preferably occupies 20% or more, and more preferably 30% or more of the area of the floor surface and the side surface from the floor surface to a height of 50 cm in the inner surface of the housing chamber 11. Although not particularly limited, the copper layer may occupy 10% or more and 100% or less or 98% or less of the area of the floor surface and the side surface from the floor surface to a height of 50 cm in the inner surface of the housing chamber 11.

In this embodiment, the copper layer 20 made of copper or a copper alloy is preferably also provided on at least a part of an outer surface of the housing chamber 11.

Here, in this embodiment, the above-described copper layer 20 is preferably provided so as to occupy 10% or more of the total area of the outer surface of the housing chamber 11.

The above-described copper layer 20 preferably occupies 20% or more, and more preferably 30% or more of the total area of the outer surface of the housing chamber 11. Although not particularly limited, the upper limit of the proportion of the copper layer 20 in the total area of the outer surface may be 100% or less. In addition, the upper limit of the Cu content of the copper or copper alloy constituting the copper layer 20 on the outer surface of the housing chamber 11 may be 99.999 mass% or less, or 75 mass% or less.

The above-described copper layer 20 may be provided by being formed at a necessary position in the inner surface (and outer surface) of the load carrying platform (housing chamber) of the existing livestock transportation vehicle.

The method of forming the copper layer 20 is not particularly limited, and a copper plate may be attached by welding, screwing, adhesion, or the like, or may be formed by plating or the like. Since the load carrying platform (housing chamber) of the existing livestock transportation vehicle is made of, for example, aluminum, an aluminum alloy, stainless steel, common steel, or the like, the method of forming the copper layer 20 is preferably selected in consideration of the bondability between the above materials and the copper layer.

In addition, in this embodiment, the thickness of the copper layer 20 is preferably in a range of 1 µm or more and less than 10 mm.

In a case where the thickness of the copper layer 20 is 1 µm or more, the copper layer 20 is not easily worn, and the antibacterial action and the antiviral action can be obtained for a certain period of time. In addition, since copper has a high density, an excessive increase of the weight of the livestock transportation container 10 can be suppressed by adjusting the thickness of the copper layer 20 to less than 10 mm.

The lower limit of the thickness of the copper layer 20 is preferably 30 µm or more, and more preferably 80 µm or more. The upper limit of the thickness of the copper layer 20 is preferably 5 mm or less, and more preferably 2 mm or less.

As the copper or copper alloy constituting the copper layer 20, any copper material having an antibacterial action and an antiviral action by copper is applicable.

Here, the copper or copper alloy constituting the copper layer 20 preferably has a Cu content of 40 mass% or more. By securing the Cu content, it is possible to surely secure an antibacterial action and an antiviral action by copper.

The Cu content in the copper or copper alloy constituting the copper layer 20 is more preferably 45 mass% or more, and even more preferably 50 mass% or more. Although not particularly limited, the copper or copper alloy constituting the copper layer 20 may have a Cu content of 40 mass% or more and 100 mass% or less. In addition, the upper limit of the Cu content of the copper or copper alloy constituting the copper layer 20 may be 99.999 mass% or less, or 75 mass% or less.

As the copper or copper alloy constituting the copper layer 20, those having an Ag content in a range of 5 mass ppm or more and 1 mass% or less may be applied.

Ag has an antibacterial action and an antiviral action by being ionized. Accordingly, it is possible to obtain an antibacterial action and an antiviral action by Ag ions in a moist use environment. Meanwhile, since Ag is expensive, the cost increases as its content increases. Therefore, as described above, the Ag content is preferably specified.

Here, in order to surely obtain the antibacterial action and the antiviral action by Ag ions, the lower limit of the Ag content is preferably 7 mass ppm or more, and more preferably 10 mass ppm or more. In order to further suppress the cost, the upper limit of the Ag content is preferably 0.7 mass% or less, and more preferably 0.5 mass% or less.

In a case where Ag is contained as impurities without being intentionally added, the Ag content may be less than 5 mass ppm.

Furthermore, as the copper or copper alloy constituting the copper layer 20, a copper alloy having a composition having a Cu content in a range of 52 mass% or more and 56 mass% or less, an Ni content in a range of 10 mass% or more and 12 mass% or less, a Mn content in a range of 0.1 mass% or more and 1.0 mass% or less, and a Zn balance containing inevitable impurities may be used.

The copper alloy having the above-described composition has excellent antibacterial properties and antiviral properties, and good workability and color fastness, and is particularly suitable as a material of the above-described copper layer 20. In addition, the copper alloy also has excellent weldability, and by welding a copper plate, the copper layer 20 can be formed. Furthermore, the copper alloy has a yellowish silvery-white color, and is excellent in appearance.

In addition, as the copper or copper alloy constituting the copper layer 20, a copper alloy having a composition having a Cu content in a range of 69 mass% or more and 73 mass% or less, an Ni content in a range of 1.5 mass% or more and 3.5 mass% or less, a Sn content in a range of 0.3 mass% or more and 0.9 mass% or less, a P content in a range of 0.01 mass% or more and 0.03 mass% or less, and a Zn balance containing inevitable impurities may be used.

The copper alloy having the above-described composition has excellent antibacterial properties and antiviral properties, and good workability and color fastness, and is particularly suitable as a material of the above-described copper layer 20. In addition, the copper alloy also has excellent weldability, and by welding a copper plate, the copper layer 20 can be formed. Furthermore, the copper alloy has a slightly whitish brass color, and is excellent in appearance.

Furthermore, in the above-described copper layer 20, the average crystal grain size is preferably less than 10 mm.

Corrosion resistance can be improved by adjusting the average crystal grain size to less than 10 mm, and it is possible to suppress the early corrosion of the copper layer 20 by excretion or the like.

The average crystal grain size of the copper layer 20 is more preferably less than 2 mm, and even more preferably less than 1 mm.

In the above-described copper layer 20, the surface roughness is preferably in a range of 0.8 or more and 25 or less. Here, the above-described surface roughness is Ra specified in JIS B0601-2001.

In a case where the surface roughness is 0.8 or more, the antibacterial action and the antiviral action can be effectively exerted. In a case where the surface roughness is 25 or less, the stress of the livestock in a case where the livestock touches the copper layer is reduced.

The lower limit of the surface roughness of the copper layer 20 is more preferably 1.6 or more. The upper limit of the surface roughness of the copper layer 20 is more preferably 12.5 or less.

According to the livestock transportation container 10 according to this embodiment having the above-described configuration, since the copper layer 20 made of copper or a copper alloy having a high action speed on pathogens and having excellent antibacterial properties and antiviral properties is provided on at least a part of the inner surface of the housing chamber 11 in which livestock is housed, it is possible to suppress the spread of pathogens to the livestock in the housing chamber 11. Accordingly, it is possible to stably transport the livestock while maintaining a good health status of the livestock.

In addition, in this embodiment, in a case where the copper layer 20 is provided so as to occupy 10% or more of the total area of the inner surface of the housing chamber 11, pathogens can be efficiently killed, and it is possible to further suppress the spread of pathogens to the livestock in the housing chamber 11.

Furthermore, in this embodiment, in a case where the copper layer 20 made of copper or a copper alloy is provided on at least a part of the outer surface of the housing chamber 11, even pathogens such as bacteria and viruses adhering to the outer surface of the housing chamber 11 can be rapidly killed by the antibacterial action and the antiviral action of the copper layer 20, and it is possible to sufficiently suppress the invasion of pathogens from the outside.

In addition, in this embodiment, in a case where the copper layer 20 is provided so as to occupy 10% or more of the total area of the outer surface of the housing chamber 11, pathogens adhering to the outer surface of the housing chamber 11 can be efficiently killed, and it is possible to further suppress the invasion of pathogens from the outside.

Furthermore, in this embodiment, in a case where the copper layer 20 is provided so as to occupy 10% or more of the area of the floor surface in the inner surface of the housing chamber 11, pathogens can be killed by the antibacterial action and the antiviral action of the copper layer 20 even in a case where pigs or the like touch tips of their noses to the floor surface, and it is possible to further suppress the spread of the pathogens.

Alternatively, in this embodiment, in a case where the copper layer 20 is provided so as to occupy 10% or more of the area of the floor surface and the side surface from the floor surface to a height of 50 cm in the inner surface of the housing chamber 11, pathogens can be killed by the antibacterial action and the antiviral action of the copper layer 20 even in a case where pigs or the like touch tips of their noses to the floor surface or the region in the side surface from the floor surface to a height of 50 cm, and it is possible to further suppress the spread of the pathogens.

Furthermore, in this embodiment, in a case where the thickness of the copper layer 20 is in a range of 1 µm or more and less than 10 mm, the antibacterial properties and the antiviral properties can be maintained for a certain period of time. In addition, an excessive increase of the weight of the copper layer 20 can be suppressed, and it is thus possible to reduce the weight of the livestock transportation container 10.

Furthermore, in this embodiment, in a case where the Cu content of the copper layer 20 is 40 mass% or more, the Cu content in the copper layer 20 is secured, and the antibacterial action and the antiviral action can be sufficiently exerted. Accordingly, it is possible to suppress the spread of pathogens in the housing chamber 11.

In addition, in this embodiment, in a case where the Ag content of the copper layer 20 is in a range of 5 mass ppm or more and 1 mass% or less, it is possible to obtain the antibacterial action and the antiviral action by eluted Ag ions in a moist use environment, and it is possible to further improve the antibacterial properties and the antiviral properties. In addition, the content of expensive Ag can be suppressed, and the manufacturing cost can be reduced.

Furthermore, in this embodiment, in a case where the copper layer 20 is made of a copper alloy having a composition having a Cu content in a range of 52 mass% or more and 56 mass% or less, an Ni content in a range of 10 mass% or more and 12 mass% or less, a Mn content in a range of 0.1 mass% or more and 1.0 mass% or less, and a Zn balance containing inevitable impurities, particularly excellent antibacterial properties and antiviral properties are obtained, and it is possible to further suppress the spread of pathogens. In addition, deterioration of appearance can be suppressed even after long-term use due to excellent color fastness.

In addition, in this embodiment, in a case where the copper layer 20 is made of a copper alloy having a composition having a Cu content in a range of 69 mass% or more and 73 mass% or less, an Ni content in a range of 1.5 mass% or more and 3.5 mass% or less, a Sn content in a range of 0.3 mass% or more and 0.9 mass% or less, a P content in a range of 0.01 mass% or more and 0.03 mass% or less, and a Zn balance containing inevitable impurities, particularly excellent antibacterial properties and antiviral properties are obtained, and it is possible to further suppress the spread of pathogens. In addition, deterioration of appearance can be suppressed even after long-term use due to excellent color fastness.

The embodiments of the present invention have been described as above, but the present invention is not limited thereto, and can be appropriately changed without departing from the technical ideas of the present invention.

For example, in this embodiment, the livestock transportation container applied as a load carrying platform of the livestock transportation vehicle shown in Fig. 1 has been described, but the present invention is not limited thereto, and may be used for a livestock transportation vehicle having another structure.

In addition, a livestock transportation container which is used in the transportation of livestock by other means for transportation such as a ship, an aircraft, or a train rather than the livestock transportation vehicle may be provided.

### [Examples]

Hereinafter, results of confirmation experiments performed to confirm the effects of the present invention will be described.

First, copper plates (thickness 3 mm) made of copper or a copper alloy shown in Table 1 were prepared. In Examples of the present invention, a copper layer was formed by attaching the copper plate to a surface of an iron sheet made of common steel.

In Comparative Example 1, the surface of an iron sheet made of common steel was exposed. In Comparative Example 2, a photocatalyst layer (titanium oxide film: thickness 1 µm) was formed on the surface of an iron sheet made of common steel.

Antibacterial characteristics were evaluated using the test pieces of the Examples of the present invention and Comparative Examples. The evaluation results are shown in Table 1.

The test method for antibacterial characteristics was performed based on JIS Z 2801. Stainless steel sheets having no copper layer were used as the test pieces and control (Comparative Example 1). A certain amount of Staphylococcus aureus was inoculated on the surface of each test piece, and the viable cell number (cfu) was quantified over time. In the test using the titanium oxide film as Comparative Example 2, ultraviolet rays were applied. A sterilization rate (log cfu/4 cm2) obtained from the following expression using the quantified viable cell numbers was taken as the antibacterial characteristics.

Sterilization Rate = log (viable cell number [cfu] of each test piece at each time/viable cell number [cfu] of stainless steel sheet test piece having no copper layer at each time)

The maximum test time was 120 minutes. In the stainless steel sheet of Comparative Example 1, the viable cell number within the test time hardly changed, and the viable cell number, which was the same as in the initial state, was regarded to be constant regardless of time.

The test was performed maximum N=3 times. Since the results in most of the examples had a proportional relationship between time and sterilization rate at the beginning of the experiment, the relationship between time and sterilization rate was approximated by a linear expression in order to eliminate the influence of dispersion. However, the data in which the sterilization rate was less than 10-5 was not used in the calculation of the approximate expression since it was regarded to have already been completely sterilized. The sterilization rate at 0 minute was set to 0. That is, the approximate expression is a linear expression that passes through the origin.

The antibacterial characteristics in Table 1 show the sterilization rate at each time calculated from the approximate expression obtained as above.

**[Table 1]**

| | Composition of Copper plate (mass ratio) | | | | | | | Average Crystal Grain Size (mm) | Roughness Ra (µm) | Antibacterial Characteristics log (viable cell number/viable cell number (no copper plate)) | | | | |
|---|---|---|---|---|---|---|---|---|---|---|---|---|---|---|
| | Cu (%) | Ag (ppm) | Ni (%) | Mn (%) | Sn (%) | P (%) | Zn | | | | | | | |
| | | | | | | | | | | 0 Minute | 15 Minutes | 30 Minutes | 60 Minutes | 120 Minutes |
| Example 1 | >99.99 | 1.00 | - | - | - | - | - | <1 | 6.3 | 0.00 | -1.24 | -2.48 | -4.96 | <-5 |
| Example 2 | >99.99 | 5.00 | - | - | - | - | - | <1 | 6.3 | 0.00 | -1.26 | -2.51 | <-5 | <-5 |
| Example 3 | >99.99 | 7.00 | - | - | - | - | - | <1 | 6.3 | 0.00 | -1.26 | -2.52 | <-5 | <-5 |
| Example 4 | >99.99 | 10.00 | - | - | - | - | - | <1 | 6.3 | 0.00 | -1.27 | -2.54 | <-5 | <-5 |
| Example 5 | 40.00 | 10.00 | - | - | - | - | balance | <1 | 6.3 | 0.00 | -0.90 | -1.80 | -3.60 | <-5 |
| Example 6 | 40.00 | 1.00 | - | - | - | - | balance | <1 | 6.3 | 0.00 | -0.90 | -1.79 | -3.58 | <-5 |
| Example 7 | 45.00 | 6.00 | - | - | - | - | balance | <1 | 6.3 | 0.00 | -0.92 | -1.83 | -3.66 | <-5 |
| Example 8 | 50.00 | 8.00 | - | - | - | - | balance | <1 | 6.3 | 0.00 | -0.95 | -1.89 | -3.78 | <-5 |
| Example 9 | 52.00 | 8.00 | 10 | 0 | - | - | balance | <1 | 6.3 | 0.00 | -1.03 | -2.05 | -4.10 | <-5 |
| Example 10 | 56.00 | 8.00 | 12 | 1 | - | - | balance | <1 | 6.3 | 0.00 | -1.04 | -2.07 | -4.14 | <-5 |
| Example 11 | 69.00 | 10.00 | 2 | - | 0 | 0 | balance | <1 | 6.3 | 0.00 | -1.12 | -2.23 | -4.46 | <-5 |
| Example 12 | 73.00 | 10.00 | 4 | - | 1 | 0 | balance | <1 | 6.3 | 0.00 | -1.13 | -2.25 | -4.50 | <-5 |
| Example 13 | >99.99 | 10.00 | - | - | - | - | - | 1.0 | 6.3 | 0.00 | -1.27 | -2.53 | <-5 | <-5 |
| Example 14 | >99.99 | 10.00 | - | - | - | - | - | 1.9 | 6.3 | 0.00 | -1.26 | -2.52 | <-5 | <-5 |
| Example 15 | >99.99 | 10.00 | - | - | - | - | - | <1 | 0.8 | 0.00 | -1.22 | -2.44 | -4.88 | <-5 |
| Example 16 | >99.99 | 10.00 | - | - | - | - | - | <1 | 1.6 | 0.00 | -1.24 | -2.47 | -4.94 | <-5 |
| Comparative Example 1 | no copper plate | | | | | | | <1 | 6.3 | 0.00 | 0.00 | 0.00 | 0.00 | 0.00 |
| Comparative Example 2 | photocatalyst layer (titanium oxide film) | | | | | | | <1 | 6.3 | 0.00 | -0.53 | -1.05 | -2.10 | -4.20 |

In Comparative Example 1 in which no copper plate (copper layer) was disposed, the viable cell number did not change even after a long period of time, and no antibacterial effect was recognized. Therefore, it has been confirmed that it was not possible to suppress the spread of pathogens.

In Comparative Example 2 in which the photocatalyst layer (titanium oxide film) was formed, a decrease in the viable cell number was recognized. However, it took time to kill the bacteria, and the antibacterial effect was insufficient. Therefore, it has been confirmed that it is not possible to kill pathogens at an early stage, and it is not possible to sufficiently suppress the spread of the pathogens.

On the other hand, in Examples of the present invention 1 to 16 in which the copper plate (copper layer) was disposed, it has been confirmed that the viable cell number after contact with the copper plate (copper layer) decreases in a short time, and the antibacterial effect is excellent. Therefore, it has been confirmed that it is possible to kill pathogens at an early stage, and it is possible to sufficiently suppress the spread of the pathogens.

From the above, according to the Examples of the present invention, it has been confirmed that it is possible to provide a livestock transportation container capable of suppressing the spread of pathogens such as bacteria and viruses to livestock housed in a housing chamber even in the transportation of the livestock, and maintaining a health status of the livestock to be transported.

### [Industrial Applicability]

It is possible to provide a livestock transportation container capable of suppressing the spread of pathogens such as bacteria and viruses to livestock housed in a housing chamber even in the transportation of the livestock, and maintaining a health status of the livestock to be transported.

### [Reference Signs List]

- 1:: Livestock transportation vehicle
- 3:: Transportation vehicle
- 10:: Livestock transportation container
- 11:: Housing chamber
- 12:: Floor surface
- 14:: End face portion
- 15:: Ceiling portion
- 20:: Copper layer
- X:: Cutting position of cross section along X-X

## Claims

1. A livestock transportation container which is used to house and transport livestock, the livestock transporting container comprising:
a copper layer made of copper or a copper alloy and provided on at least a part of an inner surface of a housing chamber for the livestock to be housed.

2. The livestock transportation container according to Claim 1,
wherein the copper layer is provided so as to occupy 10% or more of a total area of the inner surface.

3. The livestock transportation container according to Claim 1,
wherein the copper layer is provided so as to occupy 10% or more and 100% or less of a total area of the inner surface.

4. The livestock transportation container according to Claim 1,
wherein the copper layer has a thickness in a range of 1 µm or more and less than 10 mm.

5. The livestock transportation container according to Claim 1,
wherein the copper layer has a Cu content of 40 mass% or more.

6. The livestock transportation container according to Claim 1,
wherein the copper layer has a Cu content of 40 mass% or more and 100 mass% or less.

7. The livestock transportation container according to Claim 1,
wherein the copper layer has an Ag content in a range of 5 mass ppm or more and 1 mass% or less.

8. The livestock transportation container according to Claim 1,
wherein the copper layer has a Cu content in a range of 52 mass% or more and 56 mass% or less, an Ni content in a range of 10 mass% or more and 12 mass% or less, a Mn content in a range of 0.1 mass% or more and 1.0 mass% or less, and a Zn balance containing inevitable impurities.

9. The livestock transportation container according to Claim 8,
wherein the copper layer is provided so as to occupy 10% or more and 100% or less of a total area of the inner surface,
the copper layer has a thickness in a range of 1 µm or more and less than 10 mm, and
the copper layer has an Ag content in a range of 5 mass ppm or more and 1 mass% or less.

10. The livestock transportation container according to Claim 1,
wherein the copper layer has a Cu content in a range of 69 mass% or more and 73 mass% or less, an Ni content in a range of 1.5 mass% or more and 3.5 mass% or less, a Sn content in a range of 0.3 mass% or more and 0.9 mass% or less, a P content in a range of 0.01 mass% or more and 0.03 mass% or less, and a Zn balance containing inevitable impurities.

11. The livestock transportation container according to Claim 10,
wherein the copper layer is provided so as to occupy 10% or more and 100% or less of a total area of the inner surface,
the copper layer has a thickness in a range of 1 µm or more and less than 10 mm, and
the copper layer has an Ag content in a range of 5 mass ppm or more and 1 mass% or less.

12. The livestock transportation container according to any one of Claims 1 to 11,
wherein the copper layer is also provided on at least a part of an outer surface of the housing chamber.

13. The livestock transportation container according to Claim 12,
wherein the copper layer is provided so as to occupy 10% or more of a total area of the outer surface.

14. The livestock transportation container according to any one of Claims 1 to 11,
wherein the copper layer is provided so as to occupy 10% or more of an area of a floor surface in the inner surface.

15. The livestock transportation container according to any one of Claims 1 to 11,
wherein the copper layer is provided so as to occupy 10% or more of an area of a floor surface and a side surface from the floor surface to a height of 50 cm in the inner surface.
